# EUROPEAN PATENT APPLICATION

(11) **EP 2 006 370 A1**
(43) Date of publication of application: **24.12.2008**
(21) Application number: 08425432.5
(22) Date of filing: 18.06.2008
(51) Int. Cl.: C12M 1/12, C12Q 1/04, B01L 3/00

(54) **Device for the detection of bacteria or solid substances in a liquid sample**

(30) Priority: 19.06.2007 IT RM20070140 U
(71) Applicant: Murgia, Sergio, 09010 Uta (CA) (IT)
(72) Inventor: Murgia, Sergio, 09010 Uta (CA) (IT)
(74) Representative: Gitto, Serena

(57) **Abstract**

The present invention relates to a device (1) for detection of bacteria or solid substances in a liquid sample characterized in that it comprises a base (2), on the first face of which a dilution or reaction basin (3) and a well (4) with a hole (5) on its bottom are realized, said basin (3) and said well (4) being substantially co-planar and physically connected by a channel (6), the shape of which is such not to permit transfer of contents of the basin (3) into the well (4) when the base (2) is in a substantially horizontal position, while said shape permits full transfer of basin (3) contents into the well (4) when the device (1) is lifted from the basin (3) portion, while on the opposed face, or second face, of said basin (3) it is provided a housing for a filter (8), in correspondence of the hole (5) of the well (4), said device (1) further providing means (7) for fixing said filter (8).

## Description

The present invention relates to a device for detection of bacteria or solid substances in a liquid sample.

The present invention refers to the field of diagnostic devices realized to detect presence of bacteria or solid substances in a liquid sample, such as water, or a biologic sample, such as urine, by a colorimetric test using a filter disk and a stable reagent system, without employing instruments.

It is already available on the market a device for screening bacteria within urine samples, comprised of a kit including a disposable filter disk on the lower portion of a circular plastic plate, having on its central portion a conical well with a hole on its bottom communicating said well with filter under the plate. Laterally with respect to said well, plate is provided with housing for a small dilution cuvette, which is included in the kit as a separate element with respect to the circular plastic plate. Bacteria or solid substances detection method provides placing two droplets of urine within the dilution cuvette, placing dilution cuvette within the suitable housing of the circular plate, adding 6 droplets of a first reagent for acidification of urine within the dilution cuvette in order to make proteins precipitating and improving passage through the filter. This operation provides taking cuvette from the housing provided in the plate, pouring the cuvette contents within the well and leaving the filter absorbing urine and first reagent mixture. Then, coloration step is carried out adding 3 droplets of a second reagent within the well and letting bacteria coloring. Finally, washing phase for removal of in excess of coloring agent consists in adding 6 droplets of a third reagent within the well, letting said reagent absorbed by the filter and repeating the operation again. Result reading phase consist in observing if the filter is colored (positive test) or not (negative test) in the portion under the well.

This kind of device has many limits, mainly due to its scarce manegeability, particularly during the first phase of application of the detection method. In fact, dilution phase of urine sample with the first reagent occurs employing a small cuvette which is difficult to handle, when it must be agitated for mixing reactive with urine. This operation is important for precipitation of proteins in case of sample with Proteinuria that would clog the filter. Particularly, dilution operation is difficult mainly when wearing laboratory gloves, since cuvette can easily drop from hands, and, dropping, polluting filter or in any case requiring the use of a new device, loosing material and wasting time. Furthermore, according to the known device, operation consisting in pouring mixture from cuvette to well requires much care since mixture can very easily leak along the outer wall of the cuvette, leaking mixture on the operator fingers or, the same operator can dip his/her fingers within the well.

Problem that the present invention aims solving is that of improving manegeability of the device, mixing of substances, possibility of providing a device ensuring hygiene during application of the diagnostic method and reproducibility of results by a uniform pressure over the filter.

Therefore, object of the present invention is that of providing a device for detection of bacteria or solid substances in a liquid sample that can not only solve the above problems, but is also simple and functional, with reduced manufacturing costs.

It is therefore specific object of the present invention a device for detection of bacteria or solid substances in a liquid sample characterized in that it comprises a base, on the first face of which a dilution or reaction basin and a well with a hole on its bottom are realized, said basin and said well being substantially co-planar and physically connected by a channel, the shape of which is such not to permit transfer of contents of the basin into the well when the base is in a substantially horizontal position, while said shape permits full transfer of basin contents into the well when the device is lifted from the basin portion, while on the opposed face, or second face, of said basin it is provided a housing for a filter, in correspondence of the hole of the well, said device further providing means for fixing said filter.

According to the present invention, said channel preferably has an ascending inclination and a width decreasing from basin toward the well.

Preferably, the basin has a hemi-spherical shape, the well has a substantially conical shape and the connection channel has a frustum-conical shape with a lateral surface corresponding to the larger base tangent to the basin surface.

According to the present invention, preferably, said base has raised edges for preventing exit of biological sample and of reagents used in the diagnosis method.

According to a preferred embodiment, said means for fixing the filter are comprised of a lid, coupling above said second face of said base, said lid having means for pushing said filter, in correspondence or close the hole of said well, so as to evenly press said filter against the same hole.

Said pushing means close to the well hole can be comprised of projections provided along a circumference which is concentrically outside said hole.

Further particulars and advantages will be evident from the following specification, to be read along with the enclosed drawings, wherein it is represented (for illustrative and not limitative purposes) one of the preferred embodiments of a tap, and wherein:
figure 1 shows a perspective view of the first face of the device according to the invention;
figure 2 shows a perspective view of the second face of the device according to the invention;
figure 3 shows a perspective view of the second face of the device according to the invention wherein a filter is housed and with the lid for fixing said filter;
figure 4 shows an alternative embodiment of the lid for fixing the filter.

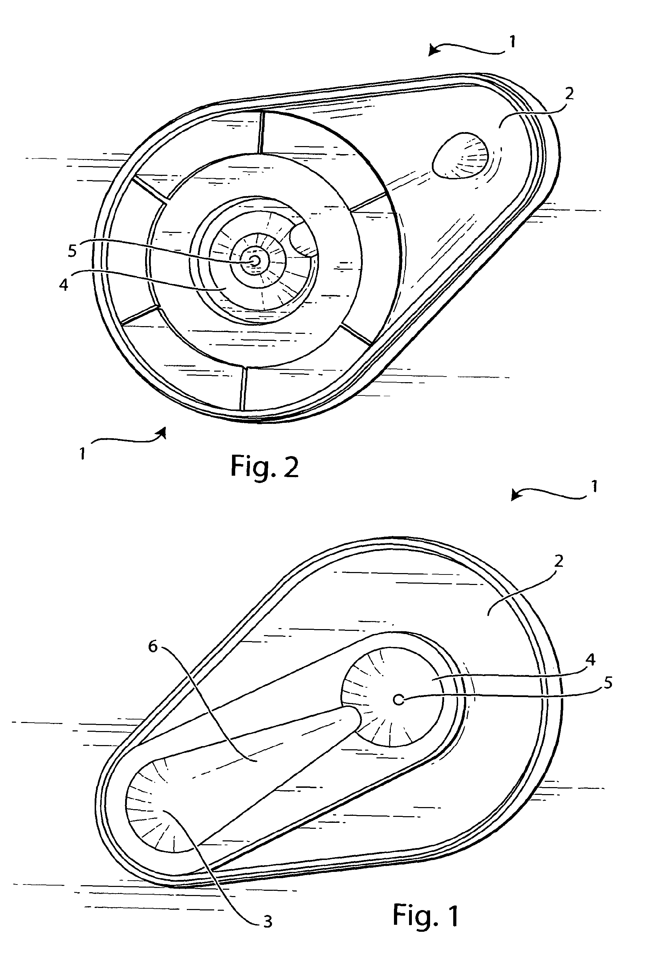

Making reference to the figures, it is shown that device 1 for detection of bacteria or solid substances in a liquid sample according to the invention is comprised of a base 2, on the first face of which (see figure 1) a dilution basin 3 and a well 4 with a hole 5 on its bottom are realized, said basin 3 and well 4 being physically connected each other by a channel 6.

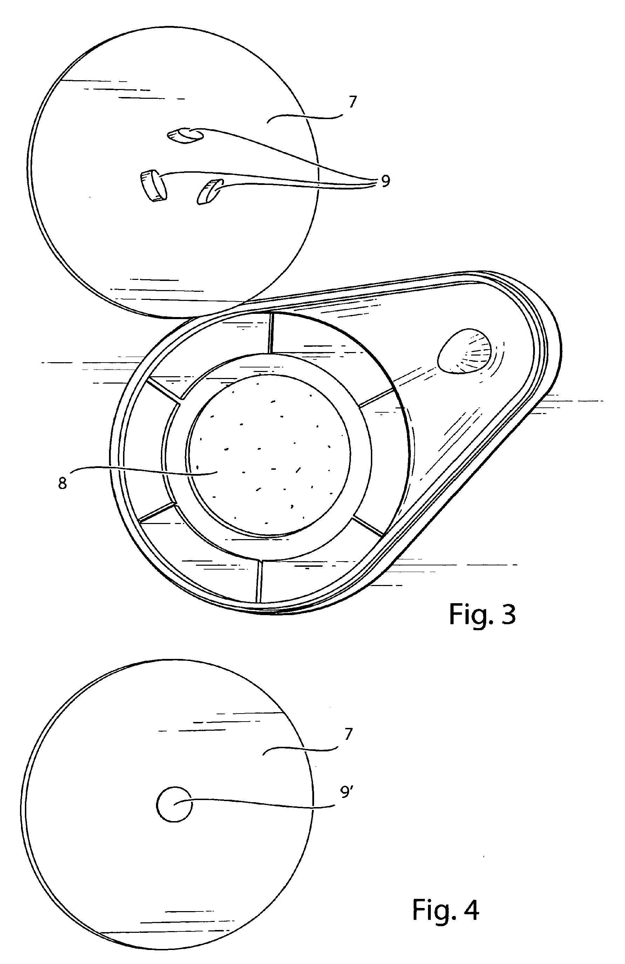

Observing particularly figures 1, 2, 3 and 4, it is noted that a filter 8 (having in the embodiment shown a disc shape, but being possible realizing it with a different shape) is placed on the other face of the base 2, said filter being fixed by a lid 7. Said lid 7 provides pushing means 9 or 9' for pushing said filter 8, respectively close (figure 3) or in correspondence (figure 4) of hole 5 of said well. Pushing means 9, close to the hole 5 of the well, are comprised of projections provided along a circumference concentrically outer with respect to the funnel hole (figure 3). Pushing means 9' in correspondence of the well hole 5 are represented by a circular projection (figure 4).

Basin 3 has a hemispherical shape, well 4 has an essentially conical shape and connection channel 6 has a frustum-conical shape with a lateral surface corresponding to the larger base which is tangent to the basin 3 surface. Said channel 6 has an ascendant inclination and width decreasing from base 3 toward well 4.

Base 2 has raised edges in order to prevent leakages of biological sample and of reagents employed in the diagnosis method.

In order to use device 1, e.g. for detection of Batteriuria, two droplets of urine are added within dilution base 3 along with 6 droplets of a first reagent for acidification of urine in order to obtain precipitation of proteins. Portion of device corresponding to basin 3 is slightly lifted so as to permit mixture flowing along channel 6 to reach well 4. Thus, mixture will be absorbed by disc filter under the well 4, exiting through hole 3 on the bottom of the same well 4. Then, the coloration step is carried out adding 3 droplets of a second reagent within well 4 and leaving filter absorbing said second reagent. Washing step for removing excess of colorant provides adding 3 droplets of a third reagent within the well 4, leaving said reagent being absorbed by filter and thus repeating again the operation. Finally, result reading step provides noting if the filter, in the portion under the well 4, is colored (positive test) or not (negative test).

In case bacteria and solid substance suspended within the water are detected, procedure provides steps of making 1 ml of sample passing through the basin 3, filtering without employing the acidifying solution N° 1 (as for examination of urine).

Second passage provides making coloration using 1 or 3 droplets of colorant. Third passage provides washing and interpreting by reading filter 8 hole 5 coloration. Diameter of filter 8 must be for this application of at least 4.5 cm. Minimum sensitivity of the system is of 100 bacteria/ml and can detect, besides bacteria, suspended substances. Device according to the invention can be also employed when it is necessary checking quality of operative water in industries, or water quality of dentist wastes, usually staying within tanks or tubes creating bactericide films. This system can be a concrete help for monitoring water quality, said water being then analyzed by other more accurate methods or sanitized by devices which are present or introduced within the system. The device can be employed in order to view also biochemical reactions in bacteria. This can be carried out soaking membrane with substrate to be metabolized and then drying reactive. Following passage provides mixing sample within the well with a physiological solution and passage within the filter with the reactive. Examples of test that can be carried out are: UREASI OXIDASE, PEPTIDASE, tripsine searching, phosphates searching, VP TEST, ecc.

Device according to the present invention has the advantage of being an efficient solution to the above mentioned problems.

In fact, since device according to the present invention provides both dilution basin and well in a single piece, connected each other by a channel, transfer of urine and first reagent mixture from dilution basin to the well is greatly simplified since it is carried out by a simple lifting of a portion of the device as described in the above without requiring handling separate accessories containing biological sample, as in the known devices. Furthermore, shape of device according to the present invention has been carefully studied so that corners are not present in correspondence of joint between dilution basin, channel and well that could retain part of the urine and first reagent mixture during the transfer of the same mixture from dilution basin to the well. Therefore, present invention permits remarkably improving manageability of device for detection of bacteria and solid substances in a liquid sample, e.g. for diagnosis of Batteriuria, simplifying diagnosis method, thus eliminating negative samples and ensuring hygiene during the method phases, preventing pouring of biologic sample or possibility that operator can dip fingers within the well.

Further, device according to the present invention is a solution which is simple and economic to be realized and managed. Among the advantages of the present invention there are included also reduction of material to be disposed of after the use of the device.

Another improvement with respect to the existing system is having studied a circular disc closing the underlying portion of the diagnostic system. If it is necessary a tangential filtering for diagnostic detection, disc has projections along a circumference which is concentrically outer with respect to the hole of the funnel (figure 3), to push and support filter by a uniform pressure in the contact zone with the funnel hole without obstructing the filtering action. In case of point colorimetric detections, disc has, instead of said projections, a circular projection (figure 4), having as well the function of pushing and supporting the filter with a uniform pressure in the contact zone with funnel filter.

Both alternative embodiments of the disc are very important in order to obtain reproducible results since they always apply the same pressure on the liquid exit point. In fact, a lower pressure permits lateral passage of liquid without tangential filtering, thus not dragging bacteria, and the result would be a false negative. Prior art system does not provide means for pushing the filter, the latter being pressed during the manufacturing and supported by a not rigid adhesive film, mainly insulating the contaminated filter.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Device (1) for detection of bacteria or solid substances in a liquid sample **characterized in that** it comprises a base (2), on the first face of which a dilution or reaction basin (3) and a well (4) with a hole (5) on its bottom are realized, said basin (3) and said well (4) being substantially co-planar and physically connected by a channel (6), the shape of which is such not to permit transfer of contents of the basin (3) into the well (4) when the base (2) is in a substantially horizontal position, while said shape permits full transfer of basin (3) contents into the well (4) when the device (1) is lifted from the basin (3) portion, while on the opposed face, or second face, of said basin (3) it is provided a housing for a filter (8), in correspondence of the hole (5) of the well (4), said device (1) further providing means (7) for fixing said filter (8).

2. Device (1) according to claim 1, **characterized in that** said channel (6) has an ascending inclination and a width decreasing from basin (3) toward the well (4).

3. Device (1) according to one of the preceding claims, **characterized in that** basin (3) has a hemi-spherical shape.

4. Device (1) according to one of the preceding claims, **characterized in that** well (4) has a substantially conical shape.

5. Device (1) according to one of the preceding claims, **characterized in that** connection channel (6) has a frustum-conical shape with a lateral surface corresponding to the larger base tangent to the basin (3) surface.

6. Device (1) according to one of the preceding claims, **characterized in that** said base (2) has raised edges for preventing exit of biological sample and of reagents used in the diagnosis method.

7. Device (1) according to one of the preceding claims, **characterized in that** said means (7) for fixing the filter (8) are comprised of a lid, coupling above said second face of said base (2), said lid having means (9) for pushing said filter (8), in correspondence of the hole (5) of said well (4), so as to evenly press said filter (8) against the same hole (5).

8. Device (1) according to one of the preceding claims 1 - 6, **characterized in that** said means (7) for fixing the filter (8) are comprised of a lid, coupling above said second face of said base (2), said lid having means (9) for pushing said filter (8), close the hole (5) of said well (4), so as to evenly press said filter (8) against the same hole (5).

9. Device (1) according to one of the preceding claims, **characterized in that** said pushing means (9') close to the well hole (5) are comprised of projections provided along a circumference which is concentrically outside said hole (5).
